# EUROPEAN PATENT APPLICATION

(11) **EP 4 792 789 A1**
(43) Date of publication of application: **19.08.2026**
(21) Application number: 26158560.8
(22) Date of filing: 13.02.2026
(51) Int. Cl.: A61F 9/06

(54) **HEADGEAR**

(30) Priority: 14.02.2025 GB 202502264
(71) Applicant: Kilworth Business and Properties Limited, Leicestershire LE17 4AT (GB)
(72) Inventor: Boffey, John, Chaffcombe, TA20 4AP (GB)
(74) Representative: Potter Clarkson

(57) **Abstract**

A headgear (20) comprises:
a welding helmet with a brow section (24); and
a respirator unit (26),
wherein the respirator unit (26) is arranged at and behind the brow section (24).

## Description

The present disclosure relates to a headgear with an in-built respirator unit.

It is known to provide a headgear to protect the face of a user during welding, grinding and cutting.

According to an aspect of the invention, there is provided a headgear comprising a welding helmet with a brow section; and a respirator unit, wherein the respirator unit is arranged at and behind the brow section.

In embodiments of the invention, the respirator unit may be attached to, or mounted on, an inward-facing side of the brow section. Such attachment or mounting may be permanent or releasable. In further embodiments of the invention, the respirator unit may be integrated with the brow section.

The respirator unit may comprise an air inlet that is arranged to face away from the brow section. Particularly, the air inlet may be arranged to face a user's head, or face rearwards of the user, when the user is wearing the headgear. The respirator unit may include a single air inlet or multiple air inlets.

The respirator unit may comprise an air outlet that is arranged to face away from the brow section. Particularly, the air outlet may be arranged to face a user's head when the user is wearing the headgear, or the air outlet may be arranged to face a direction that extends transverse to the user's head when the user is wearing the headgear. The respirator unit may include a single air outlet or multiple air outlets.

The headgear may include a breathing zone for a user wearing the headgear, wherein the air outlet may be arranged to, in use, direct air into the breathing zone, wherein the breathing zone may be sealed off from the rest of the respirator unit.

Furthermore, the respirator unit may include an air flow controller for directing air to flow through the air outlet, wherein the air flow controller may be arranged behind the brow section. Different types of air flow controllers may be employed. In a particular non-limiting example, the air flow controller may include a motor and a fan, wherein the motor is configured to, in use, drive the fan. The air flow controller and the air outlet may be configured so that, in use, air exiting the air outlet is blown across the face of a user wearing the headgear. This allows the air to circulate in front of the user's face, without blowing air directly onto the user's face which may cause discomfort.

The headgear may include a sealing member for at least partially enclosing a head of a user wearing the headgear. The sealing member may be made of a flexible material, preferably a foam material.

In embodiments of the invention, the respirator unit may include a filter. The filter may be arranged behind the brow section. Preferably the filter is arranged at the air inlet. The filter may be a replaceable or disposable filter. The respirator unit may include a single filter or multiple filters.

The headgear may include a spark guard for protecting the respirator unit. The spark guard may be arranged to cover the filter. When the respirator unit includes a filter closing mechanism, the spark guard may be arranged in or built into the filter closing mechanism.

The respirator unit may be a powered respirator unit. The headgear may further comprise a power source for supplying power to the respirator unit. The power source may be a portable power source, such as a battery, preferably a rechargeable battery. The headgear may further comprise a power source controller operable to turn the power source on and off. For example, the power source controller may be a switch for selectively forming an electrical connection between the power source and the respirator unit.

The head mount may have an adjustable size. The head mount may be a helmet, or the head mount may be a headband or harness. The head mount may be an adjustable headband with an attached helmet. The headgear may further comprise a sealing member for enclosing at least part of a head of a user wearing the headgear.

Within the scope of this application it is expressly intended that the various aspects, embodiments, examples and alternatives set out in the preceding paragraphs, and the claims and/or the following description and drawings, and in particular the individual features thereof, may be taken independently or in any combination. That is, all embodiments and all features of any embodiment can be combined in any way and/or combination, unless such features are incompatible. The applicant reserves the right to change any originally filed claim or file any new claim accordingly, including the right to amend any originally filed claim to depend from and/or incorporate any feature of any other claim although not originally claimed in that manner.

Preferred embodiments of the invention will now be described by way of non-limiting examples with reference to the following drawings, in which:
Figure 1 shows a section view of a welding headgear according to the present invention when in use;
Figure 2a shows a perspective rear view of a shield and a respirator unit of the welding headgear;
Figure 2b shows a perspective bottom view of a respirator unit of the welding headgear;
Figure 2c shows a section view of a respirator unit of the welding headgear; and
Figure a shows a welding headgear according to another embodiment of the invention.

The figures are not necessarily to scale, and certain features and certain views of the figures may be shown exaggerated in scale or in schematic form in the interests of clarity and conciseness.

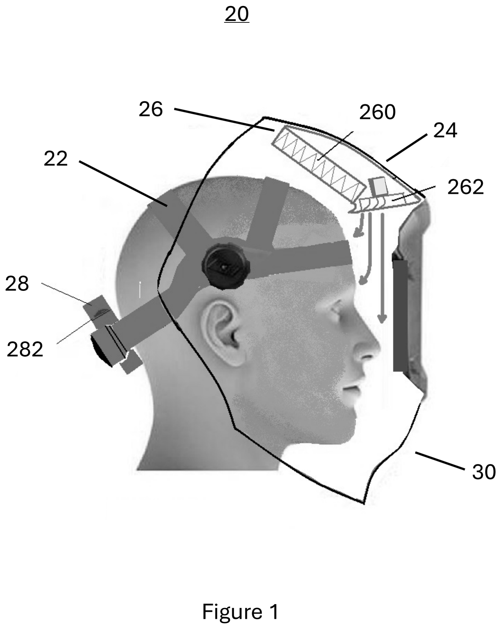

An embodiment of a welding headgear according to the present invention is shown in Figure 1 and is designated generally by the reference numeral 20.

The headgear 20 comprises a head mount 22, a welding helmet with a brow section 24, and a respirator unit 26. The head mount 22 is a headband-style head mount which is adjustable to fit securely on a head of a user. In some embodiments, the head mount 22 may be reconfigurable to allow multiple users to make use of the same headgear 20. In other embodiments, the head mount 22 may be a customised size for a single user. In further embodiments, the head mount 22 may be a harness or a belt.

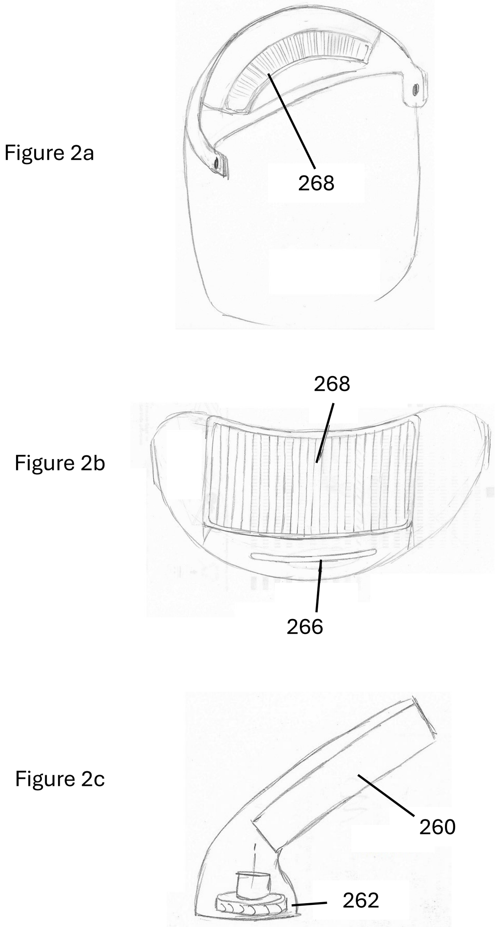

An embodiment of the respirator unit 26 is shown in Figures 2a-2c. The respirator unit 26 is arranged at and behind the brow section 24 of the head mount 22. In particular, the respirator unit 26 is mounted on an inward-facing side of the brow section 24. The respirator unit 26 comprises a filter 260 and an air flow controller that includes a motorised fan 262 driven by a motor (not shown). In the embodiment of Figure 1, the respirator unit 26 is wholly arranged at and behind the brow section 24 of the welding helmet. In other embodiments, one or more individual components of the respirator unit 26 may be located elsewhere other than at and behind the brow section. In the pictured embodiment, the fan 262 is arranged in the brow section 24 such that, in use, filtered air is blown out of an air outlet and across the face of the user. In further embodiments, the fan 262 may be arranged such that filtered air may blow in a different direction. The filter 260 filters air entering an air inlet for the safety of a user. In some embodiments it may be a replaceable filter, or it may be a refreshable filter, a reusable filter, a recyclable filter, or a disposable filter.

In some embodiments the respirator unit 26 comprises a casing, inside which the components, e.g., filter 260, fan 262 and motor, are enclosed. The casing may have several air inlets and outlets (or in some embodiments, a single air inlet and/or a single air outlet) to allow air to pass through the respirator unit 26. For example, it may have a filter closing mechanism through which a user may access the filter 260 for replacement or maintenance. It may also have a port 266 ('air outlet') through which filtered air may be blown by the fan 262 to pass across a face of a user. The port 266 directs the filtered air into a breathing zone adjacent to the face of the user. In a preferred embodiment, the breathing zone may be sealed off from the rest of the respirator unit 26.

The respirator unit 26 also comprises a spark guard 268 arranged within the filter closing mechanism 264. The spark guard 268 may prevent sparks, e.g. from welding processes and equipment, from entering the respirator unit 26 and potentially expose the filter to the risk of burning. The respirator unit 26 may further comprise an air intake to draw air from the exterior of the head mount 20 to be filtered and fed through the fan 262. The filter 260 may be a passive or unpowered filter, or it may be an active or powered filter.

Arranged on the rearward section of the head mount 22 is a power source 28. The power source 28 may be any type of portable power source, e.g., a battery, a rechargeable battery, a portable power source or portable solar cell. In further embodiments, the power source 28 may be mounted on a different portion of the headgear 20. The power source 28 is electrically connected to the respirator unit 26 and used to power the motor that drives the fan 262. In embodiments where the respirator unit 26 or headgear 20 comprises additional powered components such as an air intake or powered filter, the power source 28 may be used to power these additional powered components.

The headgear 20 may further comprise a switch or button 282 which controls the power to the respirator unit 26, or specifically to the motorised fan 262. The switch or button 282 has at least an "on" setting and an "off" setting. In some embodiments, the switch or button 282 may have additional control settings, by way of example and not limitation, the switch or button 282 may have additional settings to control the speed of the motorised fan 262. The motorised fan 262 may be used for temperature regulation or it may be used only to control flow of filtered air to the user.

The headgear 20 further comprises a welding shield 30 covering a face of a user. The welding shield 30 protects the face of the user from heat, sparks, fumes, and materials during a welding process. In some embodiments, the brow section 24 may be at least partially integrated with the shield 30. In other embodiments, the brow section 24 and the shield 30 may be separate components that are attached to each other. The respirator unit 26 may be at least partially integrated with the shield 30, or may be separate from the shield 30. The fan 262 is arranged behind a shield 30, that is, the fan is arranged inward of the shield 30 within the headgear 20.

The shield 30 may be attached to the head mount 22 by a hinge mechanism such that a user may lift the shield 30, move the shield 30 aside, or otherwise rotate the shield 30 in order to at least partially uncover their face. In other embodiments, the shield 30 may be fixedly attached to the head mount 22 such that the shield 30 must remain in position whilst a user is wearing the headgear 20.

In yet further embodiments, the shield may be capable of movement independent from the respirator unit.

The headgear may further comprise a sealing member (e.g. a rubber seal or a fabric material) for enclosing at least a head of a user. In some embodiments, the sealing apparatus and respirator unit preferably achieve a leakage of up to 2%, corresponding to a TH2 filter classification. More preferably, the leakage would be up to 0.2%, corresponding to a TH3 filter classification. The classification is preferably in accordance with British Standard EN 12941:1998+A2:2008 published on 15 April 1999.

The sealing member at least partially encloses a head of a user. In some embodiments, the sealing apparatus wholly encloses the head of a user. In other embodiments, the sealing apparatus encloses only the face of a user. In further embodiments, the sealing apparatus may enclose more than the face of a user.

The sealing member may be arranged on or around the shield of the headgear, or it may be arranged on or around the head mount of the headgear. At least part of the sealing apparatus may be permanently affixed to the headgear, or it may be completely detachable to facilitate different user requirements. The sealing member may be a flexible material such as a foam material.

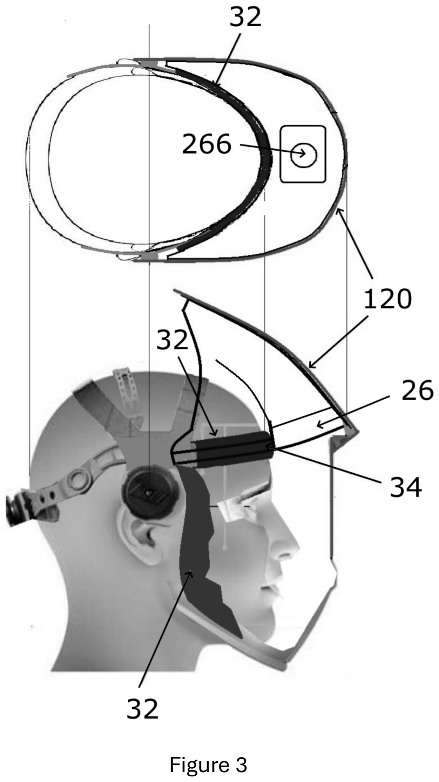

Figure 3 shows a welding headgear 120 according to another embodiment of the invention. Features of the welding headgear 20 apply mutatis mutandis to the welding headgear 120.

In the welding headgear 120, a compartment is formed behind the brow section 24. The respirator unit 26 is located in the compartment. The welding headgear 120 includes a sealing member 32 that is preferably in the form of foam material, which is arranged to provide a seal between the compartment and the breathing zone so that the breathing zone is sealed off from the respirator unit 26 except for the air outlet 266 that directs air into the breathing zone.

The foam material 32 is also arranged around the user's face, i.e. at the sides, top and/or bottom of the user's face, to seal against the outside environment. In particular, the foam material may fill a gap (or gaps) between the user's head and the headgear 120 so as to allow for a working clearance between the user's head and the headgear 120. In some embodiments, the foam material may be arranged between a head harness 34 and a body of the respirator unit 26, wherein the head harness 34 is to be worn by the user. The foam material 32 can also be used to provide comfort and sweat absorption.

In embodiments of the invention, the respirator unit 26 may have a curved shape. This allows the respirator unit 26,126 to curve around a forehead of the user for a better fit.

The configuration of the headgear according to the invention results in a lightweight headgear with in-built respiration capabilities, without requiring bulky, unwieldy and costly external components such as airducts, hoses and belts for connecting an external respirator unit to a head gear. This lightweight configuration is also more comfortable for the user. The total weight of a welding headgear according to the present invention and comprising a welding shield is preferably less than 1500 g.

Moreover, the invention provides protection not only for the user but also for the respirator unit, particularly as the filter is not exposed to the risk of burning due to being located behind the brow section. Furthermore, locating the respirator unit at and behind the brow section enables adjustment of the headgear to suit the user without the respirator unit obstructing the user.

Preferences and options for a given aspect, feature or parameter of the invention should, unless the context indicates otherwise, be regarded as having been disclosed in combination with any and all preferences and options for all other aspects, features and parameters of the invention.

The listing or discussion of an apparently prior published document or apparently prior published information in this specification should not necessarily be taken as an acknowledgement that the document is part of the state of the art or is common general knowledge.

## Claims

1. A headgear comprising:
a welding helmet with a brow section; and
a respirator unit,
wherein the respirator unit is arranged at and behind the brow section.

2. A headgear according to Claim 1 wherein the respirator unit is attached to, or mounted on, an inward-facing side of the brow section.

3. A headgear according to any one of the preceding claims wherein the respirator unit is integrated with the brow section.

4. A headgear according to any one of the preceding claims wherein the respirator unit comprises an air inlet that is arranged to face away from the brow section.

5. A headgear according to Claim 4 wherein the air inlet is arranged to face a user's head in use or face rearwards of a user in use when the user is wearing the headgear.

6. A headgear according to any one of the preceding claims wherein the respirator unit comprises an air outlet that is arranged to face away from the brow section.

7. A headgear according to Claim 6 wherein the air outlet is arranged to face a user's head in use or face a direction that extends transverse to a user's head in use when the user is wearing the headgear.

8. A headgear according to Claim 7 including a breathing zone for a user wearing the headgear, wherein the air outlet is arranged to, in use, direct air into the breathing zone, wherein the breathing zone is sealed off from the rest of the respirator unit.

9. A headgear according to any one of Claims 6 to 8 wherein the respirator unit includes an air flow controller for directing air to flow through the air outlet, wherein the air flow controller is arranged behind the brow section.

10. A headgear according to any one of the preceding claims including a sealing member for at least partially enclosing a head of a user wearing the headgear.

11. A headgear according to Claim 10 wherein the sealing member is made of a flexible material, preferably a foam material.

12. A headgear according to Claim 10 or Claim 11 wherein the sealing member and the respirator unit are arranged to, in use, at least partially enclose a head of a user wearing the headgear to achieve a TH2 classification of a maximum allowable inward leakage of up to 2% according to BS EN 12941:1998+A2:2008 published on 15 April 1999.

13. A headgear according to Claim 10 or Claim 11 wherein the sealing member and the respirator unit are arranged to, in use, at least partially enclose a head of a user wearing the headgear to achieve a TH3 classification of a maximum allowable inward leakage of up to 0.2% according to BS EN 12941:1998+A2:2008 published on 15 April 1999.

14. A headgear according to any one of the preceding claims wherein the respirator unit includes a filter, wherein the filter is arranged behind the brow section, preferably wherein the filter is arranged at the air inlet.

15. A headgear according to any one of the preceding claims including a spark guard for protecting the respirator unit, preferably wherein the spark guard is arranged to cover the filter, more preferably wherein the respirator unit includes a filter closing mechanism, wherein the spark guard is arranged in or built into the filter closing mechanism.
